# EUROPEAN PATENT APPLICATION

(11) **EP 1 219 303 A1**
(43) Date of publication of application: **03.07.2002**
(21) Application number: 00953538.6
(22) Date of filing: 21.08.2000
(51) Int. Cl.: A61K 45/00, A61K 38/40, A61K 48/00, A61K 31/7088, A61K 35/32, A61P 19/02, C07K 14/47, C07K 14/79, C12Q 1/02, G01N 33/50, G01N 33/15

(54) **CHONDROGENESIS PROMOTERS**

(30) Priority: 19.08.1999 JP 23296699
(71) Applicant: CHUGAI SEIYAKU KABUSHIKI KAISHA, Tokyo, 115-8543 (JP)
(72) Inventor: KATO, Yukio, Hiroshima-shi, Hiroshima 732-0062 (JP); FUJIMOTO, Katsumi, Hiroshima-shi, Hiroshima 734-0036 (JP)
(74) Representative: VOSSIUS & PARTNER
(86) International application number: JP0005590
(87) International publication number: WO0113951

(57) **Abstract**

There are provided a chondrogenesis stimulator containing MTf, a chondrogenic differentiation suppressing agent containing an MTf antagonist, a screening method for obtaining an MTf activating agent, an MTf activating agent obtained by the screening method, a chondrogenesis stimulator containing an MTf activating agent as obtained by the screening method, and MTf which lacks the GPI anchor region.

## Description

### TECHNICAL FIELD

This invention relates to a novel chondrogenesis stimulator. More specifically, the invention relates to a chondrogenesis stimulator containing a membrane-bound transferrin-like protein (hereunder sometimes referred to as MTf).

### PRIOR ART

The cartilage tissue of animals is composed of chondrocytes and matrix. The cartilage tissue accounts for the greater part of the skeleton at the prenatal stage and it is postnatally replaced with bone tissue due to endochondral ossification. When endochondral ossification starts, chondrocytes change from the resting to proliferating phase and the proliferating chondrocytes are then differentiated into hypertrophic chondrocytes [Reference;, "Hone no kagaku (Science of Bone)", ed. by Tsuda et al., pp. 11-29, Tokyo Ishiyaku Shuppan, 1982]. Thus, it has been well known that chondrocytes are essential cells for the formation of bone tissue, particularly at the growth stage. However, the differentiation of chondrocytes and the endochondral ossification remain unknown in many aspects.

The cell membrane of chondrocytes has characteristic glycoproteins and their membrane proteins might contribute to the unique features of chondrocytes that distinguish them from the cells of other connective tissues (as exemplified by spherical cell morphology, massive secretion of cartilage matrix, survival and proliferation in soft agar). Based on this hypothesis, Yan et al. (Yan et al.; J. Biol. Chem., vol. 265, pp. 10125-10131, 1990) and Kato et al. (Kato et al., Journal of the Society of Bone Metabolism of Japan, vol. 10, No. 2, pp. 187-192, 1992) investigated the effects of various lectins on the differentiation and proliferation of chondrocytes and, among other things, they have shown that concanavalin A (hereunder sometimes referred to as Con A) which is Jack bean lectin and which has affinity for α-D-mannose residue and α-D-glucose residue is a potent stimulator of chondrogenic differentiation, with the increase in proteoglycan synthesis being one of the criteria for the Con A activity. Chondrocytes treated with Con A change their shape from the immature flat morphology to the differentiated spherical form, inducing the production of proteoglycan and type II collagen which are markers of chondrogenic differentiation, the expression of alkaline phosphatase, etc., and even the calcification. Other lectins do not exert such differentiation inducing action.

In an attempt to search for a receptor mediating the action of Con A, Kawamoto et al. (Kawamoto et al., Eur. J. Biochem. vol. 256, pp. 503-509, 1998) paid particular attention to a protein of 76 kDa (p76) which was one of the about 20 kinds of Con A-binding proteins on chondrocytes and which would be expressed at lower levels in retinoic acid treated chondrocytes (upon treatment with retinoic acid, chondrocytes are dedifferentiated to lose reactivity with Con A). After purifying p76 from the plasma membrane fraction of rabbit chondrocytes by Con A affinity column chromatography, the N-terminal amino acid sequence was determined and the gene was cloned. In view of the determined amino acid sequence and the nucleic acid sequence of its cDNA, p76 showed 86% amino acid identity with melanotransferrin (p97) and was considered its counterpart; p97 is known as a tumor-associated antigen expressed at high levels in human tumors such as melanoma. The physiological functions of p97 are yet to be known and its expression has been reported to be high in only tumor cells, with very low detectability in normal tissue.

In view of its ability to bind with Con A, p76 is presumably involved in the differentiation of chondrocytes or in the development of their function; however, nothing has been confirmed about the effects this protein would actually impose on chondrocytes or their precursors.

An object, therefore, of the present invention is to identify a substance that will be involved in the differentiation of chondrocytes and provide a novel chondrogenesis stimulator using the substance. The present invention will lead to the invention of a substance that can control the function of chondrocytes and which eventually enables promoted osteogenesis. The substance can potentially lead to the treatment, prevention and diagnosis of new types of diseases associated with the cartilage and bone metabolisms.

### DISCLOSURE OF THE INVENTION

In order to attain the stated object, the present inventors made intensive studies and found that differentiation to cartilage could be markedly induced by overexpressing a membrane-bound transferrin-like protein (MTf) gene in mouse cell line ATDCS which retained the ability to differentiate to chondrocytes but which would hardly differentiate in the absence of stimulation.

Thus, the present invention provides a chondrogenesis stimulator containing a membrane-bound transferrin-like protein (MTf).

The MTf is preferably rabbit p76 protein, human p97 protein, mouse MTf protein, as well as a protein demonstrating the MTf activity that has an amino acid sequence encoded by DNA which hybridizes, under stringent conditions, with a DNA that encodes p76 protein or p97 protein or mouse MTf, and human p97 protein is particularly preferred.

The MTf is most preferably selected from the following:
1) a protein having the amino acid sequence of SEQ ID NO: 2;
2) a protein having the amino acid sequence of SEQ ID NO: 4;
3) a protein having the amino acid sequence of SEQ ID NO: 15; and
4) a protein demonstrating the MTf activity that has an amino acid sequence encoded by DNA which hybridizes, under stringent conditions, with a DNA encoding the protein of SEQ ID NO: 2, 4 or 15.

The present invention also provides said chondrogenesis stimulator in which the MTf lacks the GPI anchor region.

The chondrogenesis stimulator of the invention becomes more effective when used in combination with an MTf activating agent and/or insulin.

The chondrogenesis stimulator of the inventioin is useful with the following diseases: OA (osteoarthritis); RA (rheumatoid arthritis); injury of articular cartilage due to trauma; maintenance of chondrocyte phenotype in autologous transplantation of chondrocytes; reconstruction of cartilage in the ear, trachea or nose; osteochondritis dissecans; regeneration of intervetebral disk or meniscus; fractured bone; and osteogenesis from cartilage.

The invention further provides an agent for gene therapy to promote chondrogenesis which contains as an active ingredient an expression vector incorporating a DNA coding for any one of the following proteins:
1) a protein having the amino acid sequence of SEQ ID NO: 2;
2) a protein having the amino acid sequence of SEQ ID NO: 4;
3) a protein having the amino acid sequence of SEQ ID NO: 15; 4) a protein demonstrating the MTf activity that has an amino acid sequence encoded by DNA which hybridizes, under stringent conditions, with a DNA encoding the protein of SEQ ID NO: 2, 4 or 15; and
5) a protein which is the same as protein 1), 2), 3) or 4), except that it lacks the GPI anchor region.

The present invention further provides a chondrogenic differentiation suppressing agent containing an MTf antagonist.

The MTf antagonist is preferably an anti-MTf antibody or an oligonucleotide or an oligonucleotide analog that are hybridizable with a nucleic acid encoding MTf.

The present invention further provides a method for screening an MTf activating agent which comprises the steps of:
1) preparing a cell line in which MTf is overexpressed, wherein said cell line retains the ability to differentiate to chondrocytes but hardly differentiate without stimulation;
2) adding candidate substances to the cell line prepared in step 1) and culturing it for a specified period of time; and
3) examining the cell line for induced chondrogenic differentiation and selecting an MTf activating agent from the candidate substances.

The present invention also provides an MTf activating agent as obtained by the method described above.

The present invention also provides a chondrogenesis stimulator containing an MTf activating agent as obtained by the method described above.

The present invention further provides MTf which lacks the GPI anchor region.

### BRIEF DESCRIPTION OF THE DRAWINGS

Fig. 1 shows a scheme of the procedure of preparing an MTf overexpressing ATDC5 variant cells;
Fig. 2 shows the expression of the MTf gene in the variant of ATDC5 cells as analyzed by Northern blotting (photographs of electrophoresis);
Fig. 3 shows the expression of the MTf protein in the variant of ATDC5 cells as analyzed by Western blotting (photographs of electrophoresis);
Fig. 4 is a set of photographs showing that MTf overexpressing cell lines (Full-1 and Full-5) demonstrate the morphology of differentiated chondrocytes in comparison with control cells (pC-1), all of which are cultured for 29 days in the absence of insulin (biological morphology is shown);
Fig. 5 is a set of photographs showing that MTf overexpressing cell lines (Full-1 and Full-5) demonstrate the morphology of differentiated chondrocytes in comparison with control cells (pC-1), all of which are cultured for 29 days in the presence of insulin (biological, morphology is shown);
Fig. 6 is a set of photographs showing the effects of the addition of the conditioned medium of rabbit chondrocytes on the induction of chondrogenic differentiation (biological morphology is shown); and
Fig. 7 shows the result of RT-PCR Southern blotting which demonstrates the overexpression of antisense MTf RNA and the suppression of aggrecan synthesis in the presence and absence of insulin.

### BEST MODE FOR CARRYING OUT THE INVENTION

The term "membrane-bound transferrin-like protein (MTf)" as used in the invention means a protein on the cell membrane of chondrocytes that binds to Con A and which has iron-binding sites as transferrin does. Preferably, the term means a protein having the ability to mediate the induction of chondrogenic differentiation by Con A.

The term MTf has conventionally been used as the abbreviation for melanotransferrin (p97) known as a tumor antigen expressed at high levels in melanoma and other tumors. As it turned out, however, p97 is also expressed at high levels in tissues other than cancer, particularly in cartilage. Since p97 is by no means specific to cancer tissue, the present inventors redefined the term MTf as meaning "membrane-bound transferrin-like protein".

The term "MTf activity" as used herein means an activity that induces undifferentiated cells to differentiate to cartilage and which promotes chondrocytes to develop their function.

Examples of MTf include but are not limited to rabbit p76 protein, p97 protein which is a human protein homologous to rabbit p76 protein, mouse MTf protein, as well as proteins having MTf activity that contain alterations such as deletion, substitution or addition of one or more of the amino acids of these proteins, and proteins having MTf activity amino acid sequences encoded by DNA which hybridizes with DNA encoding p76 protein or p97 protein or mouse MTf protein under stringent conditions [a typical method is descried in Molecular Cloning: A Laboratory Manual, Sambrook et al., Cold Spring Harbor Laboratory Press, 1989 and consists, for example, of hybridization at 68°C in a solution containing 6 x SSC, 0.5% SDS, 10 mM EDTA, 5 x Denhardt's solution and 10 mg/ml of denatured salmon sperm DNA].

Rabbit p76 protein is homologous to human p97 protein and sometimes called rabbit p97 (Kawamoto et al., Eur. J. Biochem. vol. 256, pp. 503-509, 1998). The nucleotide and amino acid sequences of rabbit p76 protein are identified by SEQ ID NO: 1 and SEQ ID NO: 2, respectively. The nucleotide and amino acid sequences of human p97 protein are also known (Rose, T.M. et al., Pro NAS 83, 1261-1265, 1986). The nucleotide and amino acid sequences of human p97 protein are identified by SEQ ID NO: 3 and SEQ ID NO: 4, respectively. Mouse MTf protein is described in Biochim. Biophys. Acta, 1447:258-264, 1999 and its nucleotide and amino sequences are identified by SEQ ID NO: 14 and SEQ ID NO: 15, respectively. The homology between the MTf proteins over animal species are high and the amino acid identity is 83% between mouse and human, 82% between mouse and rabbit, and 86% between human and rabbit.

p76/p97 proteins are GPI anchored proteins which have glycolipid GPI (glycosylphosphatidylinositol) bound to the carboxyl group in C-terminal amino acid so that they are bound to membranes using GPI as an anchor (for p76, see Ryo Oda, Journal of Dentistry, Hiroshima University, vol. 29, No. 1, pp. 40-57, 1997; for p97, see Alemany, R. et al., J. Cell Science, 104, 1155-1162, 1993). As will be shown later in the Examples, it was verified that not only full-length MTf but also GPI anchor lacking MTf or soluble MTf have a chondrogenic differentiation inducing activity when they were expressed in non-MTf-expressing cells. Therefore, such soluble MTf, preferably the GPI anchor lacking MTf, can also be used as a chondrogenesis regulating agent. The GPI anchor lacking MTf as used herein means a soluble MTf which lacks all or part of the GPI anchor moiety; in the case of rabbit MTf, it may be exemplified by MTf in which the 28 residues at C-terminal necessary for GPI anchor binding are deleted and in the case of human MTf and mouse MTf, it may be exemplified by MTf in which the 30 residues at C-terminal necessary for GPI anchor binding are deleted.

The MTf to be used in the invention may be of a native or recombinant form and either form can be obtained by methods known in the art. The respective types of MTf are illustrated below.

### Native form

MTf can be prepared by the method described in JP 7-82297A using chondrocytes. Briefly, cartilage tissues of various animals can be used as chondrocyte source; for example, a rabbit costal growth plate cartilage as the source is treated with protease and collagenase in accordance with the method of Kato et al. (Kato et al.; J. Cell Biol., vol. 100, pp. 477-485, 1985) to obtain chondrocytes. The isolated chondrocytes can be incubated in a medium containing fetal calf serum (FCS) on a culture dish at 37°C in an atmosphere of 5% CO₂ and 95% air. The cultured chondrocytes are recovered, homogenized with a homogenizer and subjected to sedimentation equilibrium centrifugation by 17%/40% sucrose equilibrium density gradient to separate membrane proteins. The obtained membrane protein fraction is directly subjected on a concanavalin A affinity column; alternatively, in order to remove membrane proteins that bind to lectins other than concanavalin A, the membrane protein fraction is first subjected on an affinity column of wheat germ lectin which is a typical lectin and then subjected on a concanavalin A affinity column. By these and other techniques, more of the concanavalin A binding protein fraction can be separated. The specificity of the obtained concanavalin A bound protein fractions for chondrocytes can be evaluated by comparing these fractions through SDS-polyacrylamide gel electrophoresis (SDS-PAGE). After identifying the desired chondrocyte specific glycoproteins, bands of interest are excised from the gel, extracted and purified by electroelution or other suitable techniques. The resulting glycoproteins can be analyzed for the sugar chains after excising by endoglicosidase.

### Recombinant form

Recombinant MTf can be prepared by the methods described in the Examples of the invention or modifications thereof; by these methods, plasmids incorporating the MTf gene are transfected to host cells for expressing the MTf protein.

However, these are not the only methods that can be used and various methods of transformation and various host cells that are known in the art can also be used. For example, a gene encoding MTf may be inserted into a suitable vector to transform prokaryotic or eukaryotic host cells.

Further, suitable stimulators or sequences that are involved gene expression may be introduced into the vectors to enable gene expression in the transformed host cells. If desired, a gene of interest may be linked to genes encoding other polypeptides to express it as a fused protein so that it can be purified with greater ease or expressed at higher level. The desired protein can also be excised by applying suitable treatments in the purification step.

It is generally held that eukaryotic genes show polymorphism as is known for the human interferon gene. The polymorphism may cause substitution by one or more amino acids or it may cause changes in base sequences with no change occurring in amino acids.

Even polypeptides having deletion or addition of one or more amino acids within the amino acid sequence of SEQ ID NO: 2, 4 or 15 or having substitution of one or more amino acids may have a cell cycle regulating activity. For example, it is already known that a polypeptide having substitution of cysteine for serine in the human interleukin 2 (IL-2) which is derived from nucleotide alterations has exerted an IL-2 activity (Wang et al., Science 224:1431, 1984). These techniques for preparing modified genes encoding MTf protein are known to the skilled artisan.

In many cases of expression in eukaryotic cells, sugar chains may be added to the protein and the addition of sugar chains can be regulated by substituting one or more amino acids of the protein and even in this case, the chondrogenic differentiation inducing activity may be exhibited. Therefore, genes encoding such polypeptides obtained by using artificial modifications of the gene encoding MTf gene can all be used in the invention, as long as such polypeptides have the chondrogenic differentiation inducing activity.

Expression vectors that can be used include replication origins, selection markers, promoters, RNA splicing sites, polyadenylation signals and so on.

Prokaryotic organisms that can be used as host cells in the expressing system include, for example, *Escherichia coli* and *Bacillus subtilis*. Eukaryotic microorganisms that can be used as host cells include, for example, yeasts and myxomycetes. If desired, insect cells such as Sf9 may be used as host cells. Host cells derived from animal cells include, for example, COS cells and CHO cells.

Transformants thus obtained by transforming with the gene encoding MTf protein are cultured to produce proteins. The proteins can be recovered from the transformants or from the cultured medium and be purified. Not only the proteins which are obtained with genes containing nucleotide sequences encoding the amino acid sequences of SEQ ID NO: 2, 4 and 15 but also proteins which are obtained using genes containing altered nucleotide sequences encoding the amino acid sequences having substitution, deletion or addition of one or more amino acids within the amino acid sequences of SEQ ID NO: 2, 4 and 15, or proteins which are obtained using nucleotide sequences that hybridize those altered nucleotide sequences can be used as the chondrogenesis promoter of the invention as long as they have the biological function of MTf protein, namely, the chondrogenic differentiation inducing activity.

Conventional methods for separating and purifying proteins can be employed to separate and purify the MTf protein. For example, various techniques of chromatography, ultrafiltration, salting-out, dialysis, etc. can appropriately be selected and used in combination.

To use the chondrogenesis stimulator of the invention, the MTf described above may be administered in the form of a protein or it may be used an agent for gene therapy.

Insulin or an insulin-like growth factor has conventionally been known as a chondrocyte differentiating substance. It is interesting to note that the chondrogenesis stimulator of the invention induces chondrogenic differentiation even in the absence of insulin. However, it was found that the effect of the chondrogenesis stimulator is further enhanced in the presence of insulin. Therefore, the desired cartilage repairing action could be further enhanced by using MTf in combination with MTf activating agents such as insulin and an insulin-like growth factor.

When the superntant of a chondrocyte culture was added, marked differentiation of chondrocytes was observed in MTf overexpressing cell lines, suggesting that an MTf activating agent may exist in the conditioned medium of a chondrocyte culture. Therefore, the desired cartilage repairing action could be further potentiated by using MTf in combination with an MTf activating agent.

The MTf activating agent may be obtained by the following methods:
1) purifying from the conditioned medium of a chondrocyte culture system;
2) cloning the cDNA for protein binding to an MTf from a chondrocyte cDNA library; and
3) cloning the cDNA of protein binding to an MTf by the yeast two-hybrid method.

To screen various candidate substances for an MTf activating agent, a method including the following steps can be used:
1) preparing a cell line in which MTf is overexpressed, wherein said cell line retains the ability to differentiate to chondrocytes but hardly differentiate without stimulation;
2) adding candidate substances to the cell line prepared in step 1) and culturing it for a specified period of time; and
3) examining the cell line for induced chondrogenic differentiation and selecting an MTf activating agent from the candidate substances

Thus obtained MTf activating agent can be used as a chondrogenesis stimulator on its own.

It should also be mentioned that chondrogenic differentiation was induced when the soluble MTf, preferably an MTf lacking the GPI anchor region, was expressed in non-MTf-expressing cells. Therefore, the soluble MTf, preferably an MTf lacking the GPI anchor region, can be used as the chondrogenesis regulator.

The chondrogenesis stimulator of the invention can be applied to the following diseases:
1) OA (osteoarthritis);
2) RA (rheumatoid arthritis);
3) injury of articular cartilage due to trauma;
4) maintenance of chondrocyte phenotypes in autologous chondrocyte transplantation;
5) reconstruction of cartilage in the ear, trachea or nose;
6) osteochondritis dissecans;
7) regeneration of intervetebral disk or meniscus;
8) bone fracture;
9) osteogenesis from cartilage.

The chondrogenesis stimulator of the invention is generally useful as an agent for gene therapy having the MTf protein or an MTf mutant (variant) introduced in it. The agent for gene therapy of the invention contains as an active ingredient an expression vector incorporating a DNA coding for any one of the following proteins:
1) a protein having the amino acid sequence of SEQ ID NO: 2;
2) a protein having the amino acid sequence of SEQ ID NO: 4;
3) a protein having the amino acid sequence of SEQ ID NO: 15; 4) a protein demonstrating the MTf activity that has an amino acid sequence encoded by DNA which hybridizes, under stringent conditions, with a DNA encoding the protein of SEQ ID NO: 2, 4 or 15; and
5) a protein which is the same as protein 1), 2), 3) or 4), except that it lacks the GPI anchor region.

DNAs encoding MTf variants can be easily prepared by the skilled artisan using known techniques such as site-directed mutagenesis and PCR [Molecular Cloning: A Laboratory Manual, 2nd ed., Chapter 15, Cold Spring Harbor Laboratory Press (1989), and PCR - A Practical Approach, IRL Press, 200-210 (1991)].

In the present invention, an MTf- or MTf variant expression vector is provided as a DNA to be introduced into cells. These expression vectors can be prepared by linking DNA encoding an MTf- or MTf variant to an expression vector such as pSG5 (Stratagene). In the next step, the prepared DNA mixture is introduced into cells. Exemplary cells may include bone marrow interstitial cells, fibroblasts, periosteal cells, perichondral cells, synovial cells and dedifferentiated chondrocytes. DNA can be introduced into cells by the calcium phosphate method [Idenshi donyu to hatsugen kaisekiho (Gene Introduction and Methods of Expression and Analysis), ed. by Takashi Yokota and Kenichi Arai, Yodosha, 1994]. Hence, by using the introduced DNA as a medicinal active ingredient, one can prepare an agent for gene therapy which has the chondrogenesis promoting action. It is thought that, upon administering such agent for gene therapy, MTf or its variant would be expressed at high levels in cells, promoting the action of inducing chondrogenic differentiation in the cells. Therefore, the MTf containing agent of the invention for gene therapy can be used as a therapeutic or preventive of the various diseases listed above.

The agent of the invention for gene therapy can be introduced into cells by either a virus vector based method of gene introduction or a non-viral method of gene introduction [Nikkei Science, April 1994, pp. 20-45, Jikken igaku zokan (Extra Issue of Experimental Medicine), 12(15)(1994), and Jikken igaku bessatsu (Supplement to Experimental Medicine), "Idenshi chiryo no kiso gijutsu (Basic Technology in Gene Therapy)", Yodosha (1996)].

In an example of the viral vector based method of gene introduction, DNA encoding an MTf or a variant MTf is inserted into DNA or RNA viruses such as retrovirus, adenovirus, adeno-associated virus, herpes virus, vaccinia virus, poxvirus, poliovirus and Sindbis virus. Non-viral methods of gene introduction include direct intramuscular administration of an expression plasmid (DNA vaccination), liposome method, lipofectin method, microinjection, calcium phosphate method, and electroporation.

In order to ensure that the agent for gene therapy of the invention acts as a practical medicine, two methods may be used, that is, an in vivo method where DNA is directly introduced into the body and an ex vivo method where a certain kind of cells are taken out of a human and DNA is introduced into the cell, which is then put back into the body [Nikkei Science, April 1994, pp. 20-45, Gekkan yakuji (Monthly Yakuji), 36(1), 23-48 (1994), and Jikken igaku zokan (Extra Issue of Experimental Medicine), 12(15)(1994)]. The in vivo method is more preferred.

When administering the agent for gene therapy by the in vivo method, the route of administration should depend on the disease, its severity and other factors. Exemplary methods of administration include intra-articular injection, direct application to a missing part of articular cartilage, implantation (with putty, polylactic acid, etc.) and intra-articular sustained-release agent. Intravenous injection is also possible. For administration by the in vivo method, injections are generally used, with conventional carriers being added as required. Liposomes or membrane fused liposomes may be formulated as suspensions, frozen vesicles, centrifugally concentrated frozen vesicles.

In the case of the GPI anchor free soluble protein (GPI anchor lacking MTf) or the aforementioned MTf activating agent, the above-listed administrating methods may of course be used but the protein per se can be administered by various other methods. Differentiation could also be induced by adding those proteins directly to cartilage precursor cells.

The dose of the chondrogenesis stimulator of the invention is determined specifically by a doctor considering various factors such as the type of the disease to be treated, its severity, the age and body weight of the patient. The GPI anchor free soluble protein (GPI anchor lacking MTf) or the aforementioned MTf activating agent may be administered typically at a dose ranging 1 ng - 1000 mg/day, preferably 1 µg - 100 mg/day.

The present invention further provides a chondrogenic differentiation suppressing agent containing an MTf antagonist. MTf antagonists include an anti-MTf antibody, antisense DNA based on the nucleotide sequence of MTf, etc.

Antisense DNA is an oligonucleotide or an oligonucletide analog that are capable of hybridizing with an MTf coding nucleic acid.

Antisense DNA has a nucleotide sequence complementary to mRNA and forms a base pair with mRNA, thereby blocking the flow of genetic information to suppress the synthesis of MTf as the final product. The antisense DNA that can be used in the invention is an oligonucleotide capable of specifically hybridizing with a base sequence encoding the amino acid sequence identified by SEQ ID NO: 2, 4 or 15.

The term "oligonucleotide" as used herein means oligonucleotides generated from naturally occurring bases and sugar portions bound by intrinsic phosphodiester bonds, as well as analogs thereof. Therefore, the first group encompassed by this term comprises naturally occurring species or synthetic species that are generated from naturally occurring subunits or homologs thereof. The term "subunit" means a base-sugar combination which links to adjacent subunit by phosphodiester bond or other bond. The second group of oligonucleotides are their analogs that function similar to oligonucleotides but which are composed of residues having non-naturally-occurring moieties. These include oligonucleotides having chemical modifications applied to phosphate groups, sugar portions and 3'- and 5'-ends in order to provide increased stability. Examples are oligophosphorothioate and oligomethylphosphonate in which one of the oxygen atoms in the phosphodiester group between nucleotides is substituted by sulfur and -CH₃, respectively. Phosphodiester bonds may be replaced by other structures which are non-ionic and achiral. Additional oligonucleotide analogs that can be used are species containing modified base forms, that is, purine and pyrimidine in non-naturally-occurring form.

The oligonucleotides to be used in the invention have preferably 5 - 40 subunits in length, more preferably 8-30 subunits, most preferably 12- 30 subunits.

In the present invention, the target portion of mRNA with which oligonucleotides hybridize is preferably a transcription initiation site, a translation initiation site, an intron/exon junction site or a 5'-cap site; considering the secondary structure of mRNA, a site having no steric hindrance should be selected.

In the present invention, peptide nucleic acids (see, for example, Bioconjugate Chem., Vol. 5, No. 1, 1994) may be used in place of oligonucleotides.

In a particularly preferred embodiment of the invention, oligonucleotides or peptide nucleic acids that hybridize with a nucleotide sequence encoding the amino acid sequence identified by SEQ ID NO: 2 and which can inhibit MTf expression is employed.

In the present invention, oligonucleotides can be produced by synthesis methods known in the art, for example, the solid-phase synthesis method using a synthesizer as manufactured by Applied Biosystems. Similar methods can be used to produce oligonucleotide analogs such as phosphorothioate and alkylated derivatives [Akira Murakami et al., "Kinosei antisense DNA no gosei (Chemical Synthesis of Functional Antisense DNA)", Organic Synthesis Chemistry, 48(3):180-193, 1990].

The MTf antagonist that can be used in the invention is not limited to oligonucleotides of the above-defined antisense DNA providing length. To the extent that production of intrinsic MTf can be suppressed, a longer antisense, preferably an antisense of 500 - 600 nucleotides in length, may be inserted into a genome to be used for suppressing chondrogenic differentiation (see Example 3).

The anti-MTf antibody to be used in the invention is one that recognizes a peptide having at least five consecutive amino acids in the amino acid sequence identified by SEQ ID NO: 2, 4 or 15; this can be produced using a conventional procedure [see, for example, Shin-seikagaku jikken koza 1 (New Course in Biochemical Experiments 1), Protein I, pp. 389-397, 1992], which comprises immunizing an animal with an antigenic peptide having at least five consecutive amino acids in the amino acid sequence of SEQ ID NO: 2, 4 or 15, isolating the antibody produced in the animal body, and purifying the isolated antibody. The antibody may include a polyclonal and a monoclonal antibody and methods of preparing these antibodies are also known to the skilled artisan.

The following examples are provided to further illustrate the present invention but are in no way to be taken as limiting the invention. Various alterations and modifications can be made by the skilled artisan and are included within the scope of the invention.

### Examples

### Materials and Methods of Experiment

### Rabbit chondrocyte culture

Chondrocytes were isolated from rabbit costal cartilage using, with necessary modifications, the method of Kato et al. (Kato et al.: J. Cell Biol., vol. 100, pp. 477-485, 1985). Specifically, the resting cartilage of ribs in 4-week old male Japanese albino rabbits (Hiroshima Laboratory Animals) was separated, shredded with a surgical knife and incubated in a Dulbecco's modified Eagle's medium (DMEM, Flow Laboratories) containing 8 mg/mL of actinase E (Kaken Seiyaku) and 5% fetal calf serum for 1 hour and in DMEM containing 0.15% collagenase (Worthington Biochemical) for 3 hours. Cells passing through a 120-µm nyron filter were recovered, seeded in plastic culture dishes (Corning) and grown in an alpha-modified Eagle's medium (α-MEM, Sanko Junyaku) containing 10% fetal calf serum (Mitsubishi Kasei), 50 µg/mL of ascorbic acid, 50 U/mL of G potassium, 60 µg/mL of kanamycin (all being from Meiji Seika) and 250 µg/mL of amphotericin B (ICN Biochemical) (medium A) or in a serum-free DMEM (medium B) at 37°C in the atmosphere of 5% CO₂ gas.

### Mouse chondrogenic cell line ATDC5 culture

ATDC5 was purchased from Riken Cell Bank (Tsukuba, Japan). The cells were cultured in a 1:1 mixture of Ham F-12 medium (Flow Laboratories) and a Dulbecco's modified Eagle's medium (DMEM, Flow Laboratories) containing 5% fetal calf serum (FCS, Mitsubishi Kasei), 10 µg/mL of human transferrin (Boehringer Mannheim) and 0.3 nmol/mL of sodium selenite (Wako Junyaku) (maintenance medium) at 37°C in the atmosphere of 5% CO₂ gas. To induce cartilage differentitation, ATDC5 cells were cultured in a medium (differentiation medium) prepared by adding 10 µg/mL of bovine insulin (Sigma) to the maintenance medium.

### cDNA Cloning of rabbit MTf and nucleotide sequencing

Three days after reaching confluence, the rabbit chondrocytes were treated by the guanidine thiocyanate method to extract total RNA. On the basis of the nucleotide sequence of human MTf (Rose, T.M. et al., Pro NAS, 83, 1261-1265, 1986), a primer pair of 5'-GGCTGGAACGTGCCCGTGGGCTA-3' (forward) (SEQ ID NO: 5) and 5'-GTCCTGGGCCTTGTCCAGCAGTC-3' (reverse) (SEQ ID NO: 6) was designed and 1.5 kb MTf cDNA fragments were amplified from the total RNA (1 µg) by a reverse transcription polymerase chain reaction (RT-PCR) method. The obtained cDNA fragments were cloned into the SmaI site of pBluescript II SK vectors (Stratagene) and their nucleotide sequence was determined using Sequenase 7-deaza-dGTP DNA sequencing kit (USB). To obtain a full-length cDNA, rapid amplification of cDNA end (RACE) was performed using Marathon cDNA amplification kit (Clontech). Specifically, Marathon cDNA adapters were ligated to the both ends of the doublestranded total cDNA of rabbit chondrocytes and RACE was performed using adapter primers described above and specific primers (5'-AGAGGGACTCCGAGTATCTGGTCTC-3' (forward) (SEQ ID NO: 7) and 5'-GTCCGGCCCGACACCAACATCTTC-3' (reverse) (SEQ ID NO: 8) designed from the MTf nucleotide sequence. The amplified cDNA samples were separated in a 4.5% acrylamide gel and the principal cDNA bands were extracted from the gel and the bands were cloned into pBluescript II SK vectors. With the clones used as templates, the nucleotide sequence of full length MTf was determined using Sequenase 7-deaza-dGTP DNA sequencing kit and ABI autosequencer (ABI).

### Creating MTf overexpressing ATDC5 variant cells

Rabbit MTf cDNA (Kawamoto T. et al., EJB, 1988) of either full length or a truncated form which had the 28 residues from C-terminal necessary for GPI anchor binding deleted was inserted into pcDNA3.1/Zeo(+) plasmid expression vector (containing a cytomegalovirus very early promoter/enhancer; Invitrogen, San Diego, CA). Specifically, an EcoRI-NotI fragment including the full length was excised from a vector and inserted at the EcoRI-NotI site of pcDNA3.1/Zeo(+). To create a variant which lacks the GPI anchor binding site, a fragment was prepared having a stop codon inserted 28 amino acids upstream of the C-terminal and after confirming its sequence, the fragment was inserted at the EcoRI-NotI site of pcDNA3.1/Zeo(+).

In these ways, there were prepared a plasmid having a full length MTf cDNA (MTf Full) as an insert and a plasmid having GPI anchor-lacking MTf cDNA (MTf(-)GPI) as an insert; the two plasmids (pMTf Full and pMTf(-)GPI) were each transfected to ATDC5 cells (Riken, Tsukuba, Japan) using SuperFect Transfection Reagent (QIAGEN). By selection with Zeocin (Invitrogen), stable transformants were prepared.

Specifically, 2 x 10⁵ ATDC5 cells were seeded in 10-cm culture dishes. On the next day, 2 µg each of the plasmid DNAs to be introduced (pMTf Full and pMTf(-)GPI) and about 40 µL of SuperFect Transfection Reagent in solution were individually dissolved in a serum-free medium and stored until use, when they were rapidly mixed together and added to ATDC5 cells washed with a serum-free medium. After incubation at 37°C for 1 hour in the atmosphere of 5% CO₂ gas, a serum-supplemented medium was added and cultivation was conducted for an additional day. A control group was prepared by transfecting only the vector.

One day after the transfection, selection was started in a serum-supplemented medium containing 50 µg/mL of Zeocin and cell culture was continued for 2 weeks with medium change effected on every third day. As a result, there were obtained ATDC5 variant cell lines that would assure stable expression of MTf Full and MTf(-)GPI and these variant cell lines were subcultured in a serum-supplemented medium containing 50 µg/mL of Zeocin.

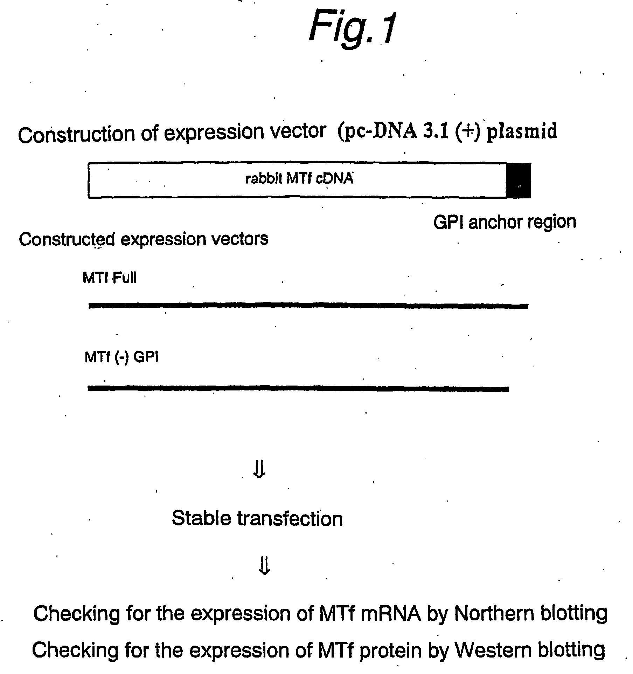

A scheme of the procedure of creating MTf overexpressing ATDC5 variant cells is shown in Fig. 1.

### Expression of rabbit MTf gene in ATDC5 variant cell lines

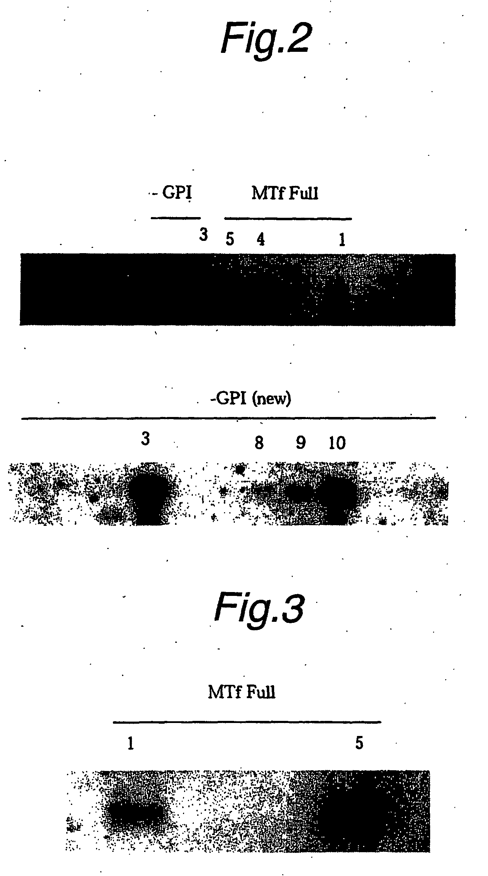

Expression of rabbit MTf gene in ATDC5 variant cell lines was confirmed by Northern blotting. Specifically, total RNA was prepared from the ATDC5 variant cell lines by the guanidine thiocyanate method; 10 µg of the total RNA was electrophoresed on a 1% agarose gel containing 2.2 mol/L of formaldehyde and transferred onto Hybond-N membrane (Amersham). The membrane was hybridized with a ³²P labeled 2.2 kb rabbit MTf cDNA probe at 42°C for 16 hours. After washing the membrane, a BioMax X-ray film (Kodak) was exposed to the membrane at -80°C to detect signals. The result is shown in Fig. 2.

In the MTf Full cell line, it was found that the rabbit MTf gene have been expressed strongly in clone Nos. 1, 4 and 5.

In the MTf(-)GPI cell line, it was found that the rabbit MTf gene have been expressed strongly in clone Nos. 3, 3N, 8, 9 and 10. In the Examples, (-)GPI-3 was used.

### Expression of rabbit MTf protein in ATDC5 variant cell lines

Expression of rabbit MTf protein in ATDC5 variant cell lines was confirmed by Western blotting. Specifically, membrane fraction protein was prepared from the ATDC5 variant cell lines, subjected to SDS-PAGE at 10 µg/lane, and transferred to a polyvinylidene difluoride membrane (Milipore). After the transfer, the membrane was blocked with 4% skimmed milk and reacted with anti-MTf serum [1:500 dilution; Eur. J. Biochem, 256, 503-509 (1988)] at 4°C for 14 hours, then reacted with ¹²⁵I sheep anti-mouse IgG(Fab')2 fragment (Amersham) at room temperature for 2 hours. The membrane was washed and a BioMax X-ray film was exposed to the membrane at -80°C for analysis. The result is shown in Fig. 3.

In the MTf Full cell line, it was found that the rabbit MTf protein have been expressed strongly in clone Nos. 1 and 5. These clones were named MTf overexpressing cell lines (Full-1 and Full-5).

### Example 1: Chondrogenic differentiation in MTf overexpressing cell lines

The MTf overexpressed cell lines (4.0 x 10⁴ cells) were seeded in 6-multiwell plates and cultured in a maintenance medium at 37°C in a 5% CO₂ gas phase.

The MTf overexpressing cell lines to be investigated were Full-1 and Full-5 which were found to have expressed both the MTf gene and protein. The MTf(-)GPI cell line to be investigated was GPI-3 which was found to have expressed the MTf gene.

As control cells, ATDC5 cells and pC-1 (vector alone) were prepared in the same manner as described above and their morphological features were examined under a microscope. Cell morphology was examined with an Olympus phase-contrast microscope. Two microscopic fields were taken for each culture system and at least 200 cells were counted to calculate the proportion of round cells.

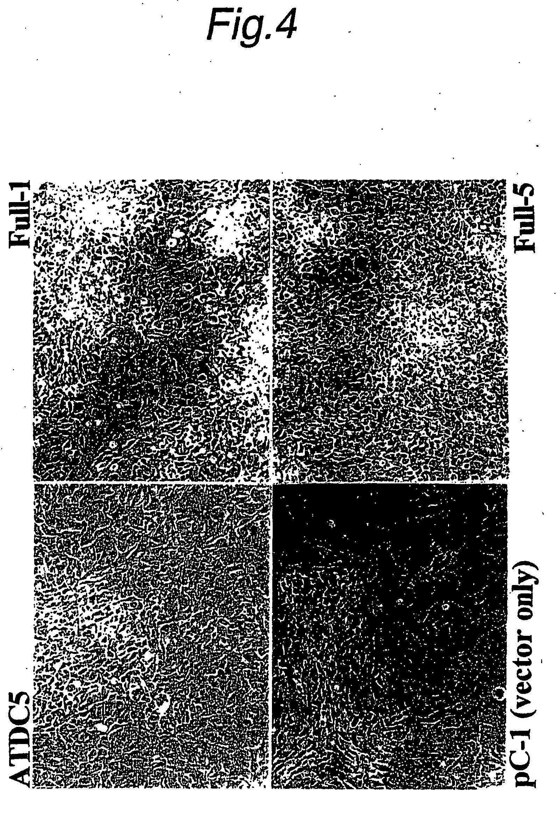

In the absence of insulin, the control cells (pC-1) did not differentiate to chondrocytes; on the other hand, the MTf overexpressing cell lines (Full-1 and Full-5) and the MTf(-)GPI cell line [(-)GPI-3] started to differentiate within 20 days and 29 days after seeding, almost all regions of the cells had differentiated to chondrocytes (Fig. 4).

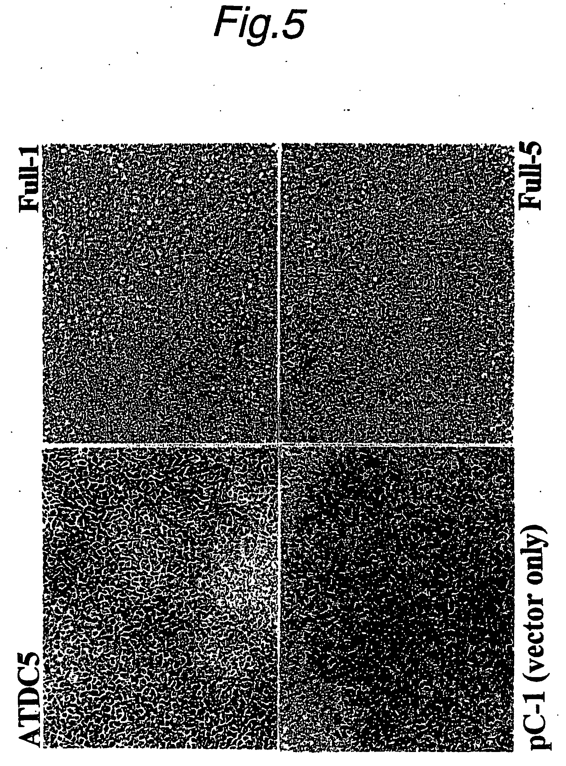

The similar test was conducted in the presence of insulin (10 µg/mL) (insulin was added at day 0). The result was the same as in the absence of insulin (Fig. 5), except that, in the presence of insulin, further differentiation was induced in the MTf overexpressing cell lines, namely, more cells "rounded" like chondrocytes than in the absence of insulin.

The above results show the effectiveness of MTf in inducing chondrogenic differentiation, which was exhibited even in the absence of insulin.

### Example 2: Effect of Adding the Conditioned Medium of Rabbit Chondrocyte Culture

Resting rabbit chondrocytes (1 x 10⁶ cells) were seeded in 10-cm culture dishes and cultured in medium A (10 mL) at 37°C in the atmosphere of 5% CO₂ gas. Two days after confluence, medium A was replaced by serum-free medium B (5 mL). After 24 hours of cell culture, the conditioned medium (CM) was recovered and subjected to an experiment. After the recovery, CM was supplemented with fetal calf serum at a concentration of 5%.

MTf overexpressing cell line (Full-5) and the control cell line (pC-1) were seeded at 8.0 x 10⁴ cells in 6-multiwell plates and cultured in a maintenance medium at 37°C in the atmosphere of 5% CO₂ gas. Three days after confluence (day 7), the previously recovered CM was added to give a concentration of 60% in the overall liquid culture; at the same time, 10 µg/mL of bovine insulin was added. Cultivation was continued for additional 48 hours at 37°C in a 5% CO₂ gas phase.

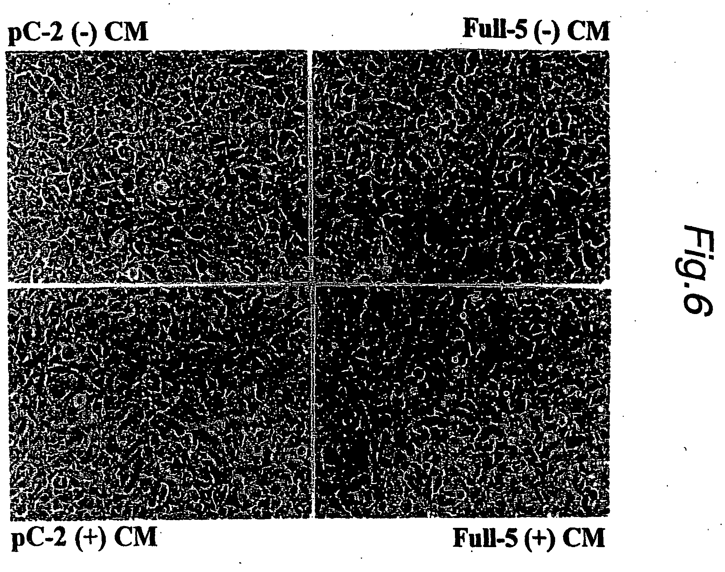

Forty-eight hours after the addition of CM, almost all cells of the MTf overexpressing cell line to which CM was added [Full-5(+)CM] differentiated to chondrocytes which synthesized active substrate and which resembled paving stones. The cells of the MTf expressing cell line to which no CM was added [Full-5(-)CM] had almost the same morphology as the control cell lines in which only a vector was expressed [pC-2(+)CM and (-)CM]. The cell lines in which only a vector was expressed had no visible induced differentiation to chondrocytes due to the addition of CM (Fig. 6).

These results show the presence of an MTf activating agent in CM.

### Example 3: Chondrogenic differentiation due to Overexpression of Antisense MTf RNA

ATDC5 variant cell lines (A-01, A-05, A-08, A-09, A-11, A-12, A-23 and A-24) in which mouse antisense MTf RNA was overexpressed were prepared by the similar method used in preparing the ATDC5 variant cell lines in which MTf was forcibly expressed. The mouse antisense MTf RNA was prepared as follows: cDNA fragments of mouse MTf were amplified by PCR (using primers 5'-GGTGTGTTGAGGGGCGTGGACTCT-3' (SEQ ID NO: 9) and 5'-TCACCAACGGCTTTGAGCACATCAC-3' (SEQ ID NO: 10), inserted into pGEM-T Easy Vector (Promega), excised with ApaI-NotI and inserted into pcDNA3.1/Zeo(+) at ApaI-NotI site (i.e., inserted in reverse direction). The sequence of the inserted portion is identified by SEQ ID NO: 11.

A control group was also prepared by transfecting only the vector (pC-1).

The expression of mouse MTf antisense was examined by Northern blotting using the above-mentioned ApaI-NotI fragment as a probe.

A criterion for the suppression of chondrogenic differentiation was the suppression of synthesis of a cartilage proteoglycan, aggrecan, and a test was conducted by RT-PCR Southern blotting both in the presence of insulin (added in an amount of 10 µg/mL after day 4) and in its absence. Specifically, total RNA was extracted from the cells of each clone by the guanidine thiocyanate method; single-stranded cDNA was synthesized from the extracted total RNA (1 µg) using SUPERSCRIPT pre-amplification system kit (Life Technologies); using the cDNA as a template, PCR was performed on the aggrecan gene with a pair of primers 5'-TGCTACTTCATCGACCC-3' (forward) (SEQ ID NO: 12) and 5'-AAAGACCTCCCCTCCATCT-3' (reverse) (SEQ ID NO: 13); the PCR reaction mixture was electrophoresed on a 1% agarose gel and transferred onto Hybond-N membrane (Amersham). The membrane was hybridized with a ³²P labeled antisense MTf probe and mouse aggrecan cDNA probe at 42°C for 16 hours. The membrane was washed with 0.2 x SSC whih contains 0.5% SDA and a BioMax X-ray film (Kodak) was exposed to the membrane at -80°C to detect signals.

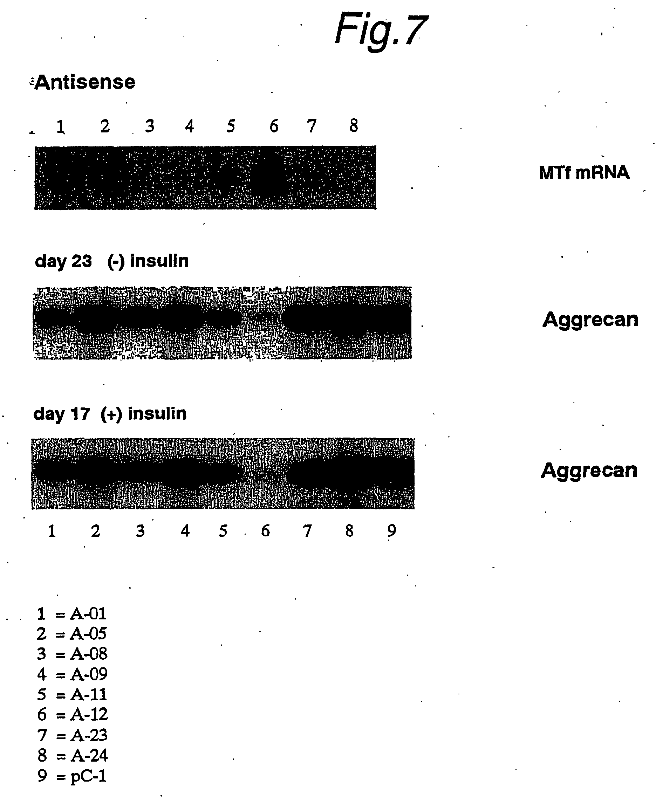

The result is shown in Fig. 7. Antisense MTf RNA was expressed most strongly in variant cell line A-12 (lane 6), followed by A-11 (lane 5) in strength. Correspondingly, expression of the aggrecan gene was suppressed most effectively in variant cell line A-12 (lane 6), followed by A-11 (lane 5) in effectiveness. While expression of the aggrecan gene was suppressed in the absence of insulin, it was more effectively suppressed in the presence of insulin.

## Claims

1. A cartridge formation stimulator containing a membrane-bound transferrin-like protein (MTf).

2. The chondrogenesis stimulator according to claim 1, wherein MTf is selected from the group consisting of rabbit p76 protein, human p97 protein, and a protein demonstrating the MTf activity that has an amino acid sequence encoded by DNA which hybridizes, under stringent conditions, with a DNA coding for p76 protein or p97 protein.

3. The chondrogenesis stimulator according to claim 1, wherein the MTf is selected from the following:
1) a protein having the amino acid sequence of SEQ ID NO: 2;
2) a protein having the amino acid sequence of SEQ ID NO: 4;
3) a protein having the amino acid sequence of SEQ ID NO: 15; and
4) a protein demonstrating the MTf activity that has an amino acid sequence encoded by DNA which hybridizes, under stringent conditions, with a DNA encoding the protein of SEQ ID NO: 2, 4 or 15.

4. The chondrogenesis stimulator according to claim 2, wherein the MTf is human p97 protein.

5. A chondrogenesis stimulator containing soluble MTf.

6. The chondrogenesis stimulator according to claim 5, wherein the soluble MTf lacks the GPI anchor region.

7. An agent for gene therapy to promote chondrogenesis which contains as an active ingredient an expression vector incorporating a DNA encoding any one of the following proteins:
1) a protein having the amino acid sequence of SEQ ID NO: 2;
2) a protein having the amino acid sequence of SEQ ID NO: 4;
3) a protein having the amino acid sequence of SEQ ID NO: 15; 4) a protein demonstrating the MTf activity that has an amino acid sequence encoded by DNA which hybridizes, under stringent conditions, with a DNA coding for the protein of SEQ ID NO: 2, 4 or 15; and
5) a protein which is the same as protein 1), 2), 3) or 4), except that it lacks the GPI anchor region.

8. The chondrogenesis stimulator according to claim 1 which is used in combination with an MTf activating agent.

9. The chondrogenesis stimulator according to claim 1 which is used in combination with insulin or an insulin-like growth factor.

10. The chondrogenesis stimulator according to any one of claims 1 - 9 for treating at least one bone disease selected from the following diseases in which chondrogenic differentiation is involved: OA (osteoarthritis); RA (rheumatoid arthritis); injury of articular cartilage due to trauma; maintenance of chondrocyte phenotypes in autologous chondrocyte transplantation; reconstruction of cartilage in the ear, trachea or nose; osteochondritis dissecans; regeneration of intervetebral disk or meniscus; bone fracture; and osteogenesis from cartilage.

11. A chondrogenic differentiation suppressing agent containing an MTf antagonist.

12. The chondrogenic differentiation suppressing agent according to claim 11, wherein the MTf antagonist is an anti-MTf antibody or an oligonucleotide or an oligonucleotide analog that are hybridizable with a nucleic acid encoding MTf.

13. A method for screening an MTf activating agent which comprises the steps of:
1) preparing a cell line in which MTf is overexpressed, wherein said cell line retains the ability to differentiate to chondrocytes but hardly differentiate without stimulation;
2) adding candidate substances to the cell line prepared in step 1) and culturing it for a specified period of time; and
3) examining the cell line for induced chondrogenic differentiation and selecting an MTf activating agent from the candidate substances.

14. An MTf activating agent obtained by the method according to claim 13.

15. A chondrogenesis stimulator containing an MTf activating agent obtained by the method according to claim 13.

16. MTf which lacks the GPI anchor region.


Sequence Listing

<110> CHUGAI SEIYAKU KABUSHIKI KAISHA

<120> CHONDROGENESIS STIMULATOR

<130> YCT-501

<160> 15

<210> 1

<211> 2388

<212> cDNA

<213> Rabbit

<400> 1

gccgccgctc actcgttcgc actcggactc agacccagtc cgaccccctg gactgcgcca 60

tgcggtgccg aagcgcggct atgtggatct tcctggccct gcgcaccgca ctcggcagcg 120

tggaggtgcg gtggtgcacc gcgtccgagc ccgagcagca gaagtgcgag gacatgagcc 180

aggccttccg cgaagccggc ctccagcccg ccctgctgtg cgtgcagggc acctcggccg 240

accactgcgt ccagctcatc gcggcccacg aggccgacgc catcactctg gacggaggag 300

ccatttacga ggcggggaag gaacacggcc tgaagcccgt ggtgggcgaa gtgtatgacc 360

aagaggtggg cacctcctac tacgctgtgg ccgtggtcaa gaggagctcc aacgtgacca 420

tcaacaccct gagaggcgtg aagtcctgcc acacgggcat caaccgcacg gtgggctgga 480

acgtgcctgt gggctacctg gtggacagcg gccgcctctc agtgatgggc tgtgacgtgc 540

tcaaagcggt cagcgagtac ttcgggggca gctgcgtccc tggggcagga gagaccagat 600

actcggagtc cctctgtcgc ctctgccggg gcgacacctc cggggagggg gtgtgtgaca 660

agagccccct ggagcggtac tacgactaca gcggggcctt ccggtgcctg gcagaaggcg 720

caggggacgt ggcctttgtg aagcacagca cggtgctgga gaacacggat gggagaacac 780

tgccctcctg gggccacatg ctgatgtcac gggactttga gctgctgtgc cgggacggca 840

gccgggccag cgtcaccgag tggcagcact gccacctggc ccgggtgccc gcccacgccg 900

tggtggtccg ggccgacacc gacgcaggcc tcatcttccg gcttctcaat gagggccagc 960

ggctgttcag ccacgagggc agcagcttcc agatgttcag ctcggaggcc tacggccaga 1020

agaacctgct gttcaaagac tccacgctgg agctggtgcc catcgccaca cagacctacg 1080

aggcctggct gggccccgag tacctgcacg ccatgaaggg tctgctctgt gaccccaacc 1140

ggctgccccc atacctgcgc tggtgcgtgc tgtccacccc cgagatccag aagtgtggag 1200

acatggccgt ggccttcagc cggcagaggc tcaagccgga gatccagtgt gtctcggcgg 1260

agtcccccca gcactgcatg gagcagatcc aggctgggca catcgatgct gtgaccctga 1320

acggggagga cattcacaca gcggggaaga cttatggct gatcccggct gccggggagc 1380

tgtatgccgc ggacgacagg agtaactcgt acttcgtggt ggccgtggtg aagcgagaca 1440

gcgcctacgc cttcaccgtg gacgagctgc gcgggaagcg ctcctgccac cccggcttcg 1500

gcagcccggc cggctgggac gtcccggtgg gcgccctcat ccactggggc tacatccggc 1560

ccaggaactg cgacgtcctc acagcggtgg gtcagttctt caacgccagc tgtgtgccgg 1620

tgaacaaccc caagaagtac ccctcctcgc tgtgcgcact ctgcgtgggt gacgagcagg 1680

gccgcaacaa gtgcactggc aacagccagg agcggtacta tggcgacagt ggcgccttca 1740

ggtgcctggt ggagggtgca ggggacgtgg ccttcgtcaa gcacacgacc atctttgaca 1800

acacaaatgg ccacaatccc gagccgtggg ctgcccatct gaggagccag gactacgagc 1860

tgctgtgccc caacggcgcg cgagctgagg cgcaccagtt tgccgcctgc aacctggccc 1920

agattccgtc ccacgccgtc atggtgcggc ccgacaccaa catcttcacc gtttacggac 1980

tgctggacaa ggcccaggac ctgtttggag acgaccacaa caagaacggg ttcaagatgt 2040

tcgactcctc cagctaccac ggccgagacc tgctcttcaa ggacgccacg gtgcgcgctg 2100

tgcctgtggg cgagaggacc acctaccagg actggctggg gccggactac gtggcggctc 2160

tggaagggat gcagtcacag cggtgctcag gggcagccgt cggcgccccc ggggcctcgc 2220

tgctgccgct gctgcccctg gctgcgggcc tcctgctgtc ttcgctctga gagcagcccc 2280

gggcagcctc ggccccggca ggggagcctg cgcggaagct tcctgaacga gcccgcgccc 2340

tggctggatg tggttacctc ggcgagccgc ggggccgcgc ttccccg 2388

<210> 2

<211> 736

<212> PRT

<213> Rabbit

<400> 2

Met Arg Cys Arg Ser Ala Ala Met Trp Ile Phe Leu Ala Leu Arg Thr
1               5                   10                  15
Ala Leu Gly Ser Val Glu Val Arg Trp Cys Thr Ala Ser Glu Pro Glu
            20                  25                  30
Gln Gln Lys Cys Glu Asp Met Ser Gln Ala Phe Arg Glu Ala Gly Leu
        35                  40                  45
Gln Pro Ala Leu Leu Cys Val Gln Gly Thr Ser Ala Asp His Cys Val
    50                  55                  60
Gln Leu Ile Ala Ala His Glu Ala Asp Ala Ile Thr Leu Asp Gly Gly
65                  70                  75                  80
Ala Ile Tyr Glu Ala Gly Lys Glu His Gly Leu Lys Pro Val Val Gly
                85                  90                  95
Glu Val Tyr Asp Gln Glu Val Gly Thr Ser Tyr Tyr Ala Val Ala Val
            100                 105                 110
Val Lys Arg Ser Ser Asn Val Thr Ile Asn Thr Leu Arg Gly Val Lys
        115                 120                 125
Ser Cys His Thr Gly Ile Asn Arg Thr Val Gly Trp Asn Val Pro Val
    130                 135                 140
Gly Tyr Leu Val Asp Ser Gly Arg Leu Ser Val Met Gly Cys Asp Val
145                 150                 155                 160
Leu Lys Ala Val Ser Glu Tyr Phe Gly Gly Ser Cys Val Pro Gly Ala
                165                 170                 175
Gly Glu Thr Arg Tyr Ser Glu Ser Leu Cys Arg Leu Cys Arg Gly Asp
            180                 185                 190
Thr Ser Gly Glu Gly Val Cys Asp Lys Ser Pro Leu Glu Arg Tyr Tyr
        195                 200                 205
Asp Tyr Ser Gly Ala Phe Arg Cys Leu Ala Glu Gly Ala Gly Asp Val
    210                 215                 220
Ala Phe Val Lys His Ser Thr Val Leu Glu Asn Thr Asp Gly Arg Thr

225                 230                 235                 240

Leu Pro Ser Trp Gly His Met Leu Met Ser Arg Asp Phe Glu Leu Leu

                245                 250                 255

Cys Arg Asp Gly Ser Arg Ala Ser Val Thr Glu Trp Gln His Cys His

            260                 265                 270

Leu Ala Arg Val Pro Ala His Ala Val Val Val Arg Ala Asp Thr Asp

        275                 280                 285

Ala Gly Leu Ile Phe Arg Leu Leu Asn Glu Gly Gln Arg Leu Phe Ser

    290                 295                 300

His Glu Gly Ser Ser Phe Gln Met Phe Ser Ser Glu Ala Tyr Gly Gln

305                 310                 315                 320

Lys Asn Leu Leu Phe Lys Asp Ser Thr Leu Glu Leu Val Pro Ile Ala

                325                 330                 335

Thr Gln Thr Tyr Glu Ala Trp Leu Gly Pro Glu Tyr Leu His Ala Met

            340                 345                 350

Lys Gly Leu Leu Cys Asp Pro Asn Arg Leu Pro Pro Tyr Leu Arg Trp

        355                 360                 365

Cys Val Leu Ser Thr Pro Glu Ile Gln Lys Cys Gly Asp Met Ala Val

    370                 375                 380

Ala Phe Ser Arg Gln Arg Leu Lys Pro Glu Ile Gln Cys Val Ser Ala

385                 390                 395                 400

Glu Ser Pro Gln His Cys Met Glu Gln Ile Gln Ala Gly His Ile Asp

                405                 410                 415

Ala Val Thr Leu Asn Gly Glu Asp Ile His Thr Ala Gly Lys Thr Tyr

            420                 425                 430

Gly Leu Ile Pro Ala Ala Gly Glu Leu Tyr Ala Ala Asp Asp Arg Ser

        435                 440                 445

Asn Ser Tyr Phe Val Val Ala Val Val Lys Arg Asp Ser Ala Tyr Ala

    450                 455                 460

Phe Thr Val Asp Glu Leu Arg Gly Lys Arg Ser Cys His Pro Gly Phe
465                 470                 475                 480
Gly Ser Pro Ala Gly Trp Asp Val Pro Val Gly Ala Leu Ile His Trp
                485                 490                 495
Gly Tyr Ile Arg Pro Arg Asn Cys Asp Val Leu Thr Ala Val Gly Gln
            500                 505                 510
Phe Phe Asn Ala Ser Cys Val Pro Val Asn Asn Pro Lys Lys Tyr Pro
        515                 520                 525
Ser Ser Leu Cys Ala Leu Cys Val Gly Asp Glu Gln Gly Arg Asn Lys
    530                 535                 540
Cys Thr Gly Asn Ser Gln Glu Arg Tyr Tyr Gly Asp Ser Gly Ala Phe
545                 550                 555                 560
Arg Cys Leu Val Glu Gly Ala Gly Asp Val Ala Phe Val Lys His Thr
                565                 570                 575
Thr Ile Phe Asp Asn Thr Asn Gly His Asn Pro Glu Pro Trp Ala Ala
            580                 585                 590
His Leu Arg Ser Gln Asp Tyr Glu Leu Leu Cys Pro Asn Gly Ala Arg
        595                 600                 605
Ala Glu Ala His Gln Phe Ala Ala Cys Asn Leu Ala Gln Ile Pro Ser
    610                 615                 620
His Ala Val Met Val Arg Pro Asp Thr Asn Ile Phe Thr Val Tyr Gly
625                 630                 635                 640
Leu Leu Asp Lys Ala Gln Asp Leu Phe Gly Asp Asp His Asn Lys Asn
                645                 650                 655
Gly Phe Lys Met Phe Asp Ser Ser Ser Tyr His Gly Arg Asp Leu Leu
            660                 665                 670
Phe Lys Asp Ala Thr Val Arg Ala Val Pro Val Gly Glu Arg Thr Thr
        675                 680                 685
Tyr Gln Asp Trp Leu Gly Pro Asp Tyr Val Ala Ala Leu Glu Gly Met

    690                 695                 700

Gln Ser Gln Arg Cys Ser Gly Ala Ala Val Gly Ala Pro Gly Ala Ser

705                 710                 715                 720

Leu Leu Pro Leu Leu Pro Leu Ala Ala Gly Leu Leu Leu Ser Ser Leu

                725                 730                 735

<210> 3

<211> 2341

<212> cDNA

<213> Human

<400> 3

gcggacttcc tcggacccgg acccagcccc agcccggccc cagccagccc cgacggcgcc 60

atgcggggtc cgagcggggc tctgtggctg ctcctggctc tgcgcaccgt gctcggaggc 120

atggaggtgc ggtggtgcgc cacctcggac ccagagcagc acaagtgcgg caacatgagc 180

gaggccttcc gggaagcggg catccagccc tccctcctct gcgtccgggg cacctccgcc 240

gaccactgcg tccagctcat cgcggcccag gaggctgacg ccatcactct ggatggagga 300

gccatctatg aggcgggaaa ggagcacggc ctgaagccgg tggtgggcga agtgtacgat 360

caagaggtcg gtacctccta ttacgccgtg gctgtggtca ggaggagctc ccatgtgacc 420

attgacaccc tgaaaggcgt gaagtcctgc cacacgggca tcaatcgcac agtgggctgg 480

aacgtgcccg tgggctacct ggtggagagc ggccgcctct cggtgatggg ctgcgatgta 540

ctcaaagctg tcagcgacta ttttgggggc agctgcgtcc cgggggcagg agagaccagt 600

tactctgagt ccctctgtcg cctctgcagg ggtgacagct ctggggaagg ggtgtgtgac 660

aagagccccc tggagagata ctacgactac agcggggcct tccggtgcct ggcggaaggg 720

gcaggggacg tggcttttgt gaagcacagc acggtactgg agaacacgga tgggaagacg 780

cttccctcct ggggccaggc cctgctgtca caggacttcg agctgctgtg ccgggatggt 840

agccgggccg atgtcaccga gtggaggcag tgccatctgg cccgggtgcc tgctcacgcc 900

gtggtggtcc gggccgacac agatgggggc ctcatcttcc ggctgctcaa cgaaggccag 960

cgtctgttca gccacgaggg cagcagcttc cagatgttca gctctgaggc ctatggccag 1020

aaggatctac tcttcaaaga ctctacctcg gagcttgtgc ccatcgccac acagacctat 1080

gaggcgtggc tgggccatga gtacctgcac gccatgaagg gtctgctctg tgaccccaac 1140

cggctgcccc cctacctgcg ctggtgtgtg ctctccactc ccgagatcca gaagtgtgga 1200

gacatggccg tggccttccg ccggcagcgc ctcaagccag agatccagtg cgtgtcagcc 1260

aagtcccccc aacactgcat ggagcggatc caggctgagc aggtcgacgc tgtgacccta 1320

agtggcgagg acatttacac ggcggggaag aagtacggcc tggttcccgc agccggcgag 1380

cactatgccc cggaagacag cagcaactcg tactacgtgg tggccgtggt gagacgggac 1440

agctcccacg ccttcacctt ggatgagctt cggggcaagc gctcctgcca cgccggtttc 1500

ggcagccctg caggctggga tgtccccgtg ggtgccctta ttcagagagg cttcatccgg 1560

cccaaggact gtgacgtcct cacagcagtg agcgagttct tcaatgccag ctgcgtgccc 1620

gtgaacaacc ccaagaacta cccctcctcg ctgtgtgcac tgtgcgtggg ggacgagcag 1680

ggccgcaaca agtgtgtggg caacagccag gagcggtatt acggctaccg cggcgccttc 1740

aggtgcctgg tggagaatgc gggtgacgtt gccttcgtca ggcacacaac cgtctttgac 1800

aacacaaacg gccacaattc cgagccctgg gctgctgagc tcaggtcaga ggactatgaa 1860

ctgctgtgcc ccaacggggc ccgagccgag gtgtcccagt ttgcagcctg caacctggca 1920

cagataccac cccacgccgt gatggtccgg cccgacacca acatcttcac cgtgtatgga 1980

ctgctggaca aggcccagga cctgtttgga gacgaccaca ataagaacgg gttcaaaatg 2040

ttcgactcct ccaactatca tggccaagac ctgcttttca aggatgccac cgtccgggcg 2100

gtgcctgtcg gagagaaaac cacctaccgc ggctggctgg ggctggacta cgtggcggcg 2160

ctggaaggga tgtcgtctca gcagtgctcg ggcgcagcgg ccccggcgcc cggggcgccc 2220

ctgctcccgc tgctgctgcc cgccctcgcc gcccgcctgc tcccgcccgc cctctgagcc 2280

cggccgcccc gccccagagc tccgatgccc gcccggggag tttccgcggc ggcctctcgc 2340

gctgcggaat ccagaaggaa gctcgcga 2368

<210> 4

<211> 738

<212> PRT

<213> Human

<400> 4

Met Arg Gly Pro Ser Gly Ala Leu Trp Leu Leu Leu Ala Leu Arg Thr

1                   5                   10                  15

Val Leu Gly Gly Met Glu Val Arg Trp Cys Ala Thr Ser Asp Pro Glu

                20                  25                  30

Gln His Lys Cys Gly Asn Met Ser Glu Ala Phe Arg Glu Ala Gly Ile

            35                  40                  45

Gln Pro Ser Leu Leu Cys Val Arg Gly Thr Ser Ala Asp His Cys Val

        50                  55                  60

Gln Leu Ile Ala Ala Gln Glu Ala Asp Ala Ile Thr Leu Asp Gly Gly

65                  70                  75                  80

Ala Ile Tyr Glu Ala Gly Lys Glu His Gly Leu Lys Pro Val Val Gly

                85                  90                  95

Glu Val Tyr Asp Gln Glu Val Gly Thr Ser Tyr Tyr Ala Val Ala Val

            100                 105                 110

Val Arg Arg Ser Ser His Val Thr Ile Asp Thr Leu Lys Gly Val Lys

        115                 120                 125

Ser Cys His Thr Gly Ile Asn Arg Thr Val Gly Trp Asn Val Pro Val

    130                 135                 140

Gly Tyr Leu Val Glu Ser Gly Arg Leu Ser Val Met Gly Cys Asp Val

145                 150                 155                 160

Leu Lys Ala Val Ser Asp Tyr Phe Gly Gly Ser Cys Val Pro Gly Ala

                165                 170                 175

Gly Glu Thr Ser Tyr Ser Glu Ser Leu Cys Arg Leu Cys Arg Gly Asp

            180                 185                 190

Ser Ser Gly Glu Gly Val Cys Asp Lys Ser Pro Leu Glu Arg Tyr Tyr

        195                 200                 205

Asp Tyr Ser Gly Ala Phe Arg Cys Leu Ala Glu Gly Ala Gly Asp Val

    210                 215                 220

Ala Phe Val Lys His Ser Thr Val Leu Glu Asn Thr Asp Gly Lys Thr

225                 230                 235                 240

Leu Pro Ser Trp Gly Gln Ala Leu Leu Ser Gln Asp Phe Glu Leu Leu
                245                 250                 255
Cys Arg Asp Gly Ser Arg Ala Asp Val Thr Glu Trp Arg Gln Cys His
            260                 265                 270
Leu Ala Arg Val Pro Ala His Ala Val Val Val Arg Ala Asp Thr Asp
        275                 280                 285
Gly Gly Leu Ile Phe Arg Leu Leu Asn Glu Gly Gln Arg Leu Phe Ser
    290                 295                 300
His Glu Gly Ser Ser Phe Gln Met Phe Ser Ser Glu Ala Tyr Gly Gln
305                 310                 315                 320
Lys Asp Leu Leu Phe Lys Asp Ser Thr Ser Glu Leu Val Pro Ile Ala
                325                 330                 335
Thr Gln Thr Tyr Glu Ala Trp Leu Gly His Glu Tyr Leu His Ala Met
            340                 345                 350
Lys Gly Leu Leu Cys Asp Pro Asn Arg Leu Pro Pro Tyr Leu Arg Trp
        355                 360                 365
Cys Val Leu Ser Thr Pro Glu Ile Gln Lys Cys Gly Asp Met Ala Val
    370                 375                 380
Ala Phe Arg Arg Gln Arg Leu Lys Pro Glu Ile Gln Cys Val Ser Ala
385                 390                 395                 400
Lys Ser Pro Gln His Cys Met Glu Arg Ile Gln Ala Glu Gln Val Asp
                405                 410                 415
Ala Val Thr Leu Ser Gly Glu Asp Ile Tyr Thr Ala Gly Lys Lys Tyr
            420                 425                 430
Gly Leu Val Pro Ala Ala Gly Glu His Tyr Ala Pro Glu Asp Ser Ser
        435                 440                 445
Asn Ser Tyr Tyr Val Val Ala Val Val Arg Arg Asp Ser Ser His Ala
    450                 455                 460
Phe Thr Leu Asp Glu Leu Arg Gly Lys Arg Ser Cys His Ala Gly Phe

465                 470                 475                 480
Gly Ser Pro Ala Gly Trp Asp Val Pro Val Gly Ala Leu Ile Gln Arg
                485                 490                 495
Gly Phe Ile Arg Pro Lys Asp Cys Asp Val Leu Thr Ala Val Ser Glu
            500                 505                 510
Phe Phe Asn Ala Ser Cys Val Pro Val Asn Asn Pro Lys Asn Tyr Pro
        515                 520                 525
Ser Ser Leu Cys Ala Leu Cys Val Gly Asp Glu Gln Gly Arg Asn Lys
    530                 535                 540
Cys Val Gly Asn Ser Gln Glu Arg Tyr Tyr Gly Tyr Arg Gly Ala Phe
545                 550                 555                 560
Arg Cys Leu Val Glu Asn Ala Gly Asp Val Ala Phe Val Arg His Thr
                565                 570                 575
Thr Val Phe Asp Asn Thr Asn Gly His Asn Ser Glu Pro Trp Ala Ala
            580                 585                 590
Glu Leu Arg Ser Glu Asp Tyr Glu Leu Leu Cys Pro Asn Gly Ala Arg
        595                 600                 605
Ala Glu Val Ser Gln Phe Ala Ala Cys Asn Leu Ala Gln Ile Pro Pro
    610                 615                 620
His Ala Val Met Val Arg Pro Asp Thr Asn Ile Phe Thr Val Tyr Gly
625                 630                 635                 640
Leu Leu Asp Lys Ala Gln Asp Leu Phe Gly Asp Asp His Asn Lys Asn
                645                 650                 655
Gly Phe Lys Met Phe Asp Ser Ser Asn Tyr His Gly Gln Asp Leu Leu
            660                 665                 670
Phe Lys Asp Ala Thr Val Arg Ala Val Pro Val Gly Glu Lys Thr Thr
        675                 680                 685
Tyr Arg Gly Trp Leu Gly Leu Asp Tyr Val Ala Ala Leu Glu Gly Met
    690                 695                 700

Ser Ser Gln Gln Cys Ser Gly Ala Ala Ala Pro Ala Pro Gly Ala Pro
705                 710                 715                 720
Leu Leu Pro Leu Leu Leu Pro Ala Leu Ala Ala Arg Leu Leu Pro Pro
                725                 730                 735
Ala Leu

<210> 5
<211> 23
<212> DNA
<213> Artificial Sequence
<400> 5
ggctggaacg tgcccgtggg cta

<210> 6
<211> 23
<212> DNA
<213> Artificial Sequence
<400> 6
gtcctgggcc ttgtccagca gtc

<210> 7
<211> 25
<212> DNA
<213> Artificial Sequence
<400> 7
agagggactc cgagtatctg gtctc

<210> 8
<211> 24

<212> DNA

<213> Artificial Sequence

<400> 8

gtccggcccg acaccaacat cttc

<210> 9

<211> 24

<212> DNA

<213> Artificial Sequence

<400> 9

ggtgtgttga ggggcgtgga ctct

<210> 10

<211> 25

<212> DNA

<213> Artificial Sequence

<400> 10

tcaccaacgg ctttgagcac atcac

<210> 11

<211> 614

<212> RNA

<213> Mouse

<400> 11

tcaccaacgg ctttgagcac atcacagccc atcactgaca gatggccgct ctctacgagg   60

taaccgacag gcacgttcca gcccacagtc cggttaatgc ctgtgtggca ggacttgacg  120

cccttcaggg tgttgatggt aacattggaa ttcctcctga ccacagccac ggcataatag  180

gaagtcccaa tgtcttggtc atagacttcc cccaccactg gcttcaggcc gtgctccttc  240

cctgcctcat agatggcccc tccatccagg gtgatggcat ctgcttttg ttccttgatg  300

agctggacac agtggtcagc ggagttgccc tggacgcaga gaagggaagg acgaatgcca 360
gctccctgga aggcctcgct catgtctttg cacttctgct gctctgcgtc tgagatggta 420
caccactgca cctccatcac acagacgaca gtgcgcaggg acaggagtag ccaaaaagtc 480
acgctcagga gcctcatggc aacgttgggt tggctggggt gctggcgggt ctgtcctggc 540
ttcctcttcc ctggtctctc tggccttcac tatttaagcg cagcccgggg agagtccacg 600
cccctcaaca cacc 614

<210> 12
<211> 17
<212> DNA
<213> Artificial Sequence
<400> 12
tgctacttca tcgaccc

<210> 13
<211> 19
<212> DNA
<213> Artificial Sequence
<400> 13
aaagacctcc cctccatct

<210> 14
<211> 4158
<212> cDNA
<213> Mouse
<400> 14
ggtgtgttga ggggcgtgga ctctccccgg gctgcgctta aatagtgaag gccagagaga 60
ccagggaaga ggaagccagg acagacccgc cagcacccca gccaacccaa cgttgccatg 120
aggctcctga gcgtgacttt ttggctactc ctgtccctgc gcactgtcgt ctgtgtgatg 180

gaggtgcagt ggtgtaccat ctcagacgca gagcagcaga agtgcaaaga catgagcgag 240
gccttccagg gagctggcat tcgtccttcc cttctctgcg tccagggcaa ctccgctgac 300
cactgtgtcc agctcatcaa ggaacaaaaa gcagatgcca tcaccctgga tggaggggcc 360
atctatgagg cagggaagga gcacggcctg aagccagtgg tgggggaagt ctatgaccaa 420
gacattggga cttcctatta tgccgtggct gtggtcagga ggaattccaa tgttaccatc 480
aacaccctga agggcgtcaa gtcctgccac acaggcatta accggactgt gggctggaac 540
gtgcctgtcg gttacctcgt agagagcggc catctgtcag tgatgggctg tgatgtgctc 600
aaagccgttg gtgattattt tggaggcagc tgtgtccctg gaacaggaga aaccagccat 660
tccgagtccc tctgtcgcct ctgccgtggc gactcttctg ggcacaatgt gtgtgacaag 720
agtcccctag agagatacta cgactacagt ggagccttcc ggtgcctggc ggaaggagcc 780
ggtgacgtgg ccttcgtgaa gcacagcaca gtgctggaaa atactgatgg aaacaccctg 840
ccttcctggg gcaagtccct gatgtcagag gacttccagc tactatgcag ggatggcagc 900
cgagccgaca tcactgagtg gagacgttgc cacctggcca aggtgcctgc tcatgctgtg 960
gtggtcaggg gtgacatgga tggcggtctc atattccaac tgctcaacga aggccagctt 1020
ctgttcagcc acgaagacag cagcttccag atgttcagct ccaaagccta cagccagaag 1080
aacttgctgt tcaaagactc caccttggag cttgtgccca ttgccacaca gaactacgag 1140
gcctggctgg gccaggaata cctgcaggcc atgaaggggc tcctctgtga tcccaaccgg 1200
ctgccccact acctgcgctg gtgtgtgctg tcagcgcccg agatccagaa gtgtggagat 1260
atggctgtgg ccttcagccg ccagaatctc aagccggaaa ttcagtgtgt gtcggccgag 1320
tcccctgagc actgcatgga gcagatccag gctgggcaca ctgacgctgt gactctgagg 1380
ggcgaggaca tttacagggc aggaaaggtg tacggcctgg ttccggcggc cggggagctg 1440
tatgctgagg aggacaggag caattcctac tttgtggtgg ctgtggcaag aagggacagc 1500
tcctactcct tcaccctgga cgagcttcgc ggcaagcgtt cctgccaccc ctacttgggc 1560
agcccagcgg gctgggaggt gcccatcggc tccctcatcc agcggggctt catccggccc 1620
aaggactgtg atgtcctcac agcggtgagc cagttcttca atgccagctg cgtgcctgtc 1680
aacaacccta agaactaccc ttccgcacta tgtgcgctct gcgtgggaga cgagaagggc 1740
cgcaacaaat gtgtggggag cagccaggag agatactacg gctacagcgg ggccttcagg 1800
tgccttgtgg agcatgcagg ggacgtggct ttcgtcaagc acacgactgt ctttgagaac 1860
acaaatggtc acaatcctga gccttgggct tctcacctca ggtggcaaga ctatgaacta 1920

ctgtgcccca atggggcacg ggctgaggta gaccagttcc aagcttgcaa cctggcacaa 1980

atgccatccc acgctgtcat ggtccgtcca gacaccaaca tcttcactgt gtatggactt 2040

ctggacaagg cccaggacct gtttggagac gaccataaca agaacggttt ccaaatgttt 2100

gactcctcca aatatcacag ccaagacctg cttttcaaag atgctacagt ccgagcggtg 2160

ccagtccggg agaaaaccac atacctggac tggctgggtc ctgactatgt ggttgcgctg 2220

gaggggatgt tgtctcagca gtgctccggt gcaggggccg cggtgcagcg agtccccctg 2280

ctggccctgc tcctgctgac cctggctgca ggcctccttc ctcgcgttct ctgaagaccg 2340

ctgcttcagg ccacgcccag agcagggaaa gctacagagc tcaaccggaa gaaaccagga 2400

catcagctaa ccctgcagga gagcgcgggg cgggatgagg agaggcaagg tgagaactca 2460

cacacacaca caagcctccg aggtgcgatt ctaacccaaa gagaaatttc tagaatcagg 2520

atgattgtta aggccaagtc ttcccacttg ctggagccct caatacctga ggcgactggc 2580

gagtagccca gtcactcctc ccacaccggt ggcgccagca gcgaacctgt gcctcccacc 2640

tggagcctcc tggctggctg gggtggttaa gggggggggg gggagagtga agatgctggt 2700

tgccatggca accgtggagc agcttccagc ctctgtaccg gccacctggt gagatgccaa 2760

ggaaggagca caccaccaac ctagggaacc tgtgcgacac actaccaccc agcagcccct 2820

gctctcgctg ccccaccgct ctctcctatg ggcacttgtc caccaaggcc acaccgtcgg 2880

aggggcaagg ctgctgagca catcagcctt ctgatgtgac accaaccaag gagcccagcc 2940

ctctggacag caagattttg ctagactggg atgggaggaa ggccagagct gtactgtggg 3000

gatgaagtcc tccaaaaccc tcagaggaag gaagtgcccc caccttccca ttaagaatgt 3060

tagtgtgtga gaaacttgat gcagggtgga aactatcctg tttaacggct cccgtggcaa 3120

gcaggacttg cgctgtctgc gctgcctgga cctcactgca caatgaaact gttgccgaga 3180

ttctattgtt tgctctcctg gtctcagtct caacattagt tttctccctg ccttcatata 3240

ccccttccca catcaccacg caagcacgca cgcgcacacg cacacgcaca caccttatcc 3300

gtgtgaacat atctgaacat atctgcttgt ctgaagaagt aggagctaac ccaaaataac 3360

ttcctgtcat gagctgggcc ttgggatata ccacgagcca ggggattggg gagagccctg 3420

tcttcccttc accctgcacc tgttgggcag ttgcatcttt cgagaggatc cctggttctc 3480

tcgaactgtg agagccaagg cctaggctgc cattttgcca ttgttctctc gagaaccaga 3540

aaaagttttc caaagctacc agctcttacc ccagatcttg ttcccttaaa aaaaagtaat 3600

aaataaaaag gagaagaaac aggagcaaac agccatcgtc agcacactgg aagcagcgtg 3660

ggccgggagc tatttgtgtc ttggtctgtg tggggggcct cagatcccaa tgacaggcca 3720
ggttcccagt ggctcgcccc cacctgtggg cgacgacggg acagatcctt tccatggctc 3780
accagtagag aaggtcctgg cagtgtccca gccagagtca cacaatcctg aggaaaatcg 3840
gtcaccatgg tgcttgggag agcaagcccc tcctcctccc agtacacagc catccattct 3900
tctctgagct ggggacttca cagtgagaag tgtactctgt gtgggcgact gtgctgccca 3960
aagtgtgatg tctgtgccgt gtgcctttca ggtgtgactt tgaagagcgt tgtgtaaatg 4020
acgtctgatt gccatgggcc actgctgtgt ttgtgctaaa gaaagacatt ggtttctttt 4080
taaaataaag ccatatatcc ctgcatacgc agaggcttgg atcctggtgg aaaaaaaaaa 4140
aaaaaaaaaa aaaaaaaa 4158

<210> 15
<211> 737
<212> PRT
<213> Mouse
<400> 15